(19) Europäisches Patentamt
European Patent Office
Office européen des brevets

(11) EP 1 458 423 B1

(12) **EUROPÄISCHE PATENTSCHRIFT**

(45) Veröffentlichungstag und Bekanntmachung des Hinweises auf die Patenterteilung:
**15.02.2006 Patentblatt 2006/07**

(21) Anmeldenummer: **02799748.5**

(22) Anmeldetag: **18.12.2002**

(51) Int Cl.:
***A61L 15/24*** *(2006.01)* ***A61L 15/60*** *(2006.01)*

(86) Internationale Anmeldenummer:
**PCT/EP2002/014451**

(87) Internationale Veröffentlichungsnummer:
**WO 2003/053482 (03.07.2003 Gazette 2003/27)**

(54) **TOCOPHEROL-HALTIGE SUPERABSORBER**

SUPER-ABSORBING POLYMERS CONTAINING TOCOPHEROL

SUPERABSORBANTS CONTENANT DU TOCOPHEROL

(84) Benannte Vertragsstaaten:
**AT BE BG CH CY CZ DE DK EE ES FI FR GB GR IE IT LI LU MC NL PT SE SI SK TR**

(30) Priorität: **21.12.2001 DE 10163543**

(43) Veröffentlichungstag der Anmeldung:
**22.09.2004 Patentblatt 2004/39**

(73) Patentinhaber: **BASF Aktiengesellschaft**
**67056 Ludwigshafen (DE)**

(72) Erfinder:
- **NESTLER, Gerhard**
  **A-1070 Wien (AT)**
- **SCHRÖDER, Jürgen**
  **67071 Ludwigshafen (DE)**
- **WICKEL, Stefan**
  **67281 Bissersheim (DE)**

(56) Entgegenhaltungen:
**EP-A- 0 349 240**
**EP-A- 0 582 062**
**DE-A- 19 521 848**
**US-A- 5 278 336**
**US-A- 5 461 124**
**US-A- 5 837 789**

EP 1 458 423 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

Printed by Jouve, 75001 PARIS (FR)

**Beschreibung**

Die vorliegende Erfindung betrifft neuartige Tocopherol-haltige Superabsorber, ein Verfahren zu ihrer Herstellung unter Verwendung Tocopherol-haltiger säuregruppentragender Monomere, insbesondere Acrylsäure und ihre Verwendung zur Absorption wässriger Flüssigkeiten.

Insbesondere betrifft die vorliegende Erfindung hochquellfähige Hydrogele (Superabsorber) auf Basis Polyacrylsäure, die insbesondere alpha-Tocopherol enthalten, Verfahren zu ihrer Herstellung unter Verwendung alpha-Tocopherol-haltiger Acrylsäure, sowie deren Verwendung im Hygieneartikel.

Quellbare Hydrogel-bildende Polymerisate, sogenannte Superabsorber (Super-Absorbing Polymers), sind aus dem Stand der Technik bekannt. Hierbei handelt es sich um Netzwerke flexibler hydrophiler Polymerisate, die sowohl ionischer als auch nichtionischer Natur sein können. Diese sind in der Lage, wässrige Flüssigkeiten unter Bildung eines Hydrogels zu absorbieren und zu binden und werden daher bevorzugt für die Herstellung von Tampons, Windeln, Damenbinden, Inkontinenzartikeln, Trainingsunterwäsche für Kinder, Schuheinlagen und anderen Hygieneartikeln bei der Absorption von Körperflüssigkeiten verwendet. In den Hygieneartikeln befinden sich die Superabsorber in aller Regel im sog. absorbent core, der als weitere Materialien unter anderem Fasern (Cellulosefasern) umfasst, die als eine Art Flüssigkeitsreservoir die spontan beaufschlagten Flüssigkeitsmengen zwischenspeichern und eine gute Kanalisation der Körperflüssigkeiten im absorbent core hin zum Superabsorber gewährleisten sollen.

Der aktuelle Trend im Windelaufbau besteht darin, dünnere Konstruktionen mit reduziertem Cellulosefaseranteil und erhöhtem Hydrogelanteil herzustellen. Mit dem Trend zu immer dünner werdenden Windelkonstruktionen hat sich das Anforderungsprofil an die wasserquellbaren hydrophilen Polymeren über die Jahre deutlich verändert. Während zu Beginn der Entwicklung hochsaugfähiger Hydrogele zunächst allein das sehr hohe Quellvermögen in Vordergrund stand, hat sich später gezeigt, dass auch die Fähigkeit des Superabsorbers zur Flüssigkeitsweiterleitung und -verteilung von entscheidender Bedeutung ist. Es hat sich gezeigt, dass Superabsorber an der Oberfläche bei Benetzung mit Flüssigkeit stark anquellen, so dass der Flüssigkeitstransport ins Partikelinnere stark erschwert oder ganz unterbunden wird. Diese Eigenart des Superabsorbers wird auch als "Gelblocking" bezeichnet. Aufgrund der höheren Beladung des Hygieneartikels (Polymer pro Flächeneinheit) soll das Polymer im gequollenen Zustand keine Sperrschicht für nachfolgende Flüssigkeit bilden. Weist das Produkt gute Transporteigenschaften auf, so kann eine optimale Ausnutzung des gesamten Hygieneartikels gewährleistet werden.

Für die Verwendung der hochquellfähigen Hydrogele auf dem Hygienesektor werden derzeit Polymerisate eingesetzt, die einen Neutralisationsgrad zwischen 5 und 80 Mol%, insbesondere zwischen 60 und 80 Mol%, bezogen auf die polymerisierten, Säuregruppen enthaltenden Monomereinheiten, besitzen.

Angemessene Transporteigenschaften besitzen beispielsweise Hydrogele, die im gequollenen Zustand eine hohe Gelfestigkeit aufweisen. Gele mit nur geringer Gelfestigkeit sind unter einem angewendetem Druck (Körperdruck) deformierbar, verstopfen Poren in dem Superabsorber / Cellulosefaser-Saugkörper und verhindern dadurch eine weitere Flüssigkeitsaufnahme. Eine erhöhte Gelfestigkeit wird in aller Regel durch eine höhere Vernetzung erreicht, wodurch allerdings die Retention des Produktes verringert wird. Eine elegante Methode zur Erhöhung der Gelfestigkeit stellt die Oberflächennachvernetzung dar. Bei diesem Verfahren werden getrocknete Superabsorber mit durchschnittlicher Vernetzungsdichte einer zusätzlichen Vernetzung unterworfen. Der Prozess ist dem Fachmann bekannt und in Patent EP-A-0 349 240 beschrieben. Durch die Oberflächennachvernetzung steigt die Vernetzungsdichte in der Schale der Superabsorberpartikel, wodurch die Absorption unter Druckbelastung auf ein höheres Niveau gehoben wird. Während die Absorptionskapazität in der Superabsorberschale sinkt, weist der Kern der Superabsorberpartikel durch das Vorhandensein beweglicher Polymerketten eine im Vergleich zur Schale verbesserte Absorptionskapazität auf, so dass durch den Schalenaufbau eine verbesserte Flüssigkeitsweiterleitung gewährleistet wird, ohne dass der Effekt des Gelblockings auftritt. Es ist durchaus wünschenswert, dass die Gesamtkapazität des Superabsorbers nicht spontan, sondern zeitversetzt ausgeschöpft wird. Da der Hygieneartikel in der Regel mehrmals mit Urin beaufschlagt wird, muss das Absorptionsvermögen des Superabsorbers sinnvollerweise nicht nach der ersten Disposition erschöpft sein. Auch bei weiterer Beaufschlagung mit Flüssigkeit nimmt der Superabsorber idealerweise die Flüssigkeit zügig auf. In jedem Fall kann es bei einer starken Flüssigkeitsbelastung oder bei einer weiteren zweiten oder folgenden Flüssigkeitsbeaufschlagung zu einem Wiederaustritt der Flüssigkeit (Rewet) kommen. Tritt das Phänomen des Gelblockings aber auf, kommt es unter Umständen zum Austritt der Flüssigkeit, die zu der sog. Leakage des Hygieneartikels führt. Diese Flüssigkeit enthält auch lösliche Komponenten des Superabsorbers. Die Rücknässung (Rewet) wird durch moderne Superabsorber zwar reduziert, kann aber nicht vollständig ausgeschlossen werden.

Es bestand die Aufgabe, modifizierte Superabsorber und ein Verfahren zu deren Herstellung zur Verfügung zu stellen, die bei Verwendung z.B. in Hygieneartikeln bei einer auftretenden Rücknässung möglichst zu keiner potentiellen gesundheitlichen Belastung führen können. Außerdem bestand die Aufgabe, das Verfahren zur Herstellung von Superabsorbern zu verbessern.

Überraschenderweise wird diese Aufgabe gelöst durch den Einsatz hochquellfähiger Hydrogele, bei denen die zur Polymerisation eingesetzte Acrylsäure mit Tocopherol versetzt ist.

Gegenstand der vorliegenden Erfindung sind demnach wässrige Flüssigkeiten absorbierende Hydrogel-formende Polymere auf Basis von Polyacrylat, die Tocopherol enthalten. Bevorzugt sind wässrige Flüssigkeiten absorbierende Hydrogel-formende Polymere, bei denen das Tocopherol in dem Polymer verteilt vorliegt. Bevorzugt sind weiterhin wässrige Flüssigkeiten absorbierende Hydrogel-formende Polymere bei denen das Tocopherol alpha-Tocopherol ist. Insbesondere solche wässrige Flüssigkeiten absorbierende Hydrögelformende Polymere, bei denen das Tocopherol zwischen 10 und 1000 ppm bezogen auf säuregruppentragende Monomere, insbesondere Acrylsäure bzw. Acrylat vorliegt.

Desweiteren sind Gegenstand der vorliegenden Erfindung Verfahren zur Herstellung von wässrige Flüssigkeiten absorbierenden Hydrogel-formenden Polymeren auf Basis von Polymeren, die Säuregruppen tragen, insbesondere auf Basis von Polyacrylat, wobei die zur Herstellung verwendete säuregruppentragende Monomere, insbesondere Acrylsäure Tocopherol enthalten. Bevorzugt sind Verfahren, bei denen das Tocopherol alpha-Tocopherol ist und bei denen Tocopherol zwischen 10 und 1000 ppm bezogen auf Acrylsäure vorliegt.

Ein weiterer Gegenstand der Erfindung sind Flüssigkeit absorbierenden Hydrogel-formenden Polymeren auf Basis von säuregruppentragenden Polymeren, insbesondere Polyacrylat, erhältlich nach obigen Verfahren und deren Verwendung zur Absorption von wässrigen Flüssigkeiten, insbesondere in Hygieneartikeln.

Offenbart wird ein säuregruppentragendes Monomer, insbesondere Acrylsäure, enthaltend Tocopherol, insbesondere alpha-Tocopherol. Weiters wird offenbart ein säuregruppentragendes Monomer, insbesondere Acrylsäure, das Tocopherol als Stabilisator enthält. Bevorzugt sind mindestens 50 mol-% des Stabilisators, besonders bevorzugt mindestens 90 mol-% des Stabilisators Tocopherol, insbesondere wird nur Tocopherol als Stabilisator eingesetzt. Gegenstand der vorliegenden Erfindung ist außerdem die Verwendung von Tocopherol zur Stabilisierung von säuregruppenhaltigen Monomeren, insbesondere Acrylsäure und deren Salze.

Experimenteller Teil

Verfahren zur Herstellung

a) Eingesetzte Monomere

Hydrogel-formende Polymere sind insbesondere Polymere aus (co-)polymerisierten hydrophilen Monomeren, Pfropf(co-)polymere von einem oder mehreren hydrophilen Monomeren auf eine geeignete Pfropfgrundlage, vernetzte Cellulose- oder Stärkeether, vernetzte Carboxymethylcellulose, teilweise vernetztes Polyalkylenoxid oder in wässrigen Flüssigkeiten quellbare Naturprodukte, wie beispielsweise Guarderivate, Alginate und Carrageenane.

Geeignete Pfropfgrundlagen können natürlichen oder synthetischen Ursprungs sein. Beispiele sind Stärke, Cellulose oder Cellulosederivate sowie andere Polysacharide und Oligosacharide, Polyvinylalkohol, Polyalkylenoxide, insbesondere Polyethylenoxide und Polypropylenoxide, Polyamine, Polyamide sowie hydrophile Polyester. Geeignete Polyalkylenoxide haben beispielsweise die Formel

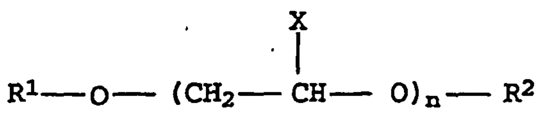

| | |
|---|---|
| $R^1$ und $R^2$ | unabhängig voneinander Wasserstoff, Alkyl, Alkenyl oder Acryl, |
| X | Wasserstoff oder Methyl und |
| n | eine ganze Zahl von 1 bis 10000 bedeuten. |

$R^1$ und $R^2$ bedeuten bevorzugt Wasserstoff, $(C_1 - C_4)$-Alkyl, $(C_2 - C_6)$-Alkenyl oder Phenyl.

Bevorzugt sind als Hydrogel-formende Polymere vernetzte Polymere mit Säuregruppen, die überwiegend in Form ihrer Salze, in der Regel Alkali- oder Ammoniumsalze, vorliegen. Derartige Polymere quellen bei Kontakt mit wässrigen Flüssigkeiten besonders stark zu Gelen auf.

Bevorzugt sind Polymere, die durch vernetzende Polymerisation oder Copolymerisation von säuregruppentragenden monoethylenisch ungesättigten Monomeren oder deren Salzen erhalten werden. Ferner ist es möglich, diese Monomere ohne Vernetzer zu (co-)polymerisieren und nachträglich zu vernetzen.

Solche Säuregruppen tragenden Monomere sind beispielsweise monoethylenisch ungesättigte $C_3$- bis $C_{25}$-Carbonsäuren oder Anhydride wie Acrylsäure, Methacrylsäure, Ethacrylsäure, α-Chloracrylsäure, Crotonsäure, Maleinsäure, Maleinsäureanhydrid, Itaconsäure, Citraconsäure, Mesaconsäure, Glutaconsäure, Aconitsäure und Fumarsäure.

Weiterhin kommen monoethylenisch ungesättigte Sulfon- oder Phosphonsäuren in Betracht, beispielsweise Vinylsulfonsäure, Allylsulfonsäure, Sulfoethylacrylat, Sulfomethacrylat, Sulfopropylacrylat, Sulfopropylmethacrylat, 2-Hydroxy-3-acryloxypropylsulfonsäure, 2-Hydroxy-3-methacryl-oxypropylsulfonsäure, Vinylphosphonsäure, Allylphosphonsäure, Styrolsulfonsäure und 2-Acrylamido-2-methylpropansulfonsäure. Die Monomeren können allein oder in Mischung untereinander eingesetzt werden.

Bevorzugt eingesetzte Monomere sind Acrylsäure, Methacrylsäure, Vinylsulfonsäure, Acrylamidopropansulfonsäure oder Mischungen dieser Säuren, z. B. Mischungen aus Acrylsäure und Methacrylsäure, Mischungen aus Acrylsäure und Acrylamidopropan-sulfonsäure oder Mischungen aus Acrylsäure und Vinylsulfonsäure. Besonders bevorzugt ist Acrylsäure. Werden in der vorliegenden Erfindung Konzentrationen in Bezug auf säuregruppentragende Monomere definiert, beziehen sich diese Konzentrationen auf die Gesamtheit der Monomeren unabhängig davon, ob die Säuregruppe protoniert oder deprotoniert vorliegt. Bezieht sich die Konzentration auf säuregruppenhaltige Polymere, bezieht sich die Konzentration auf den Gehalt an Säuregruppen in protonierter und deprotonierter Form.

Zur Optimierung von Eigenschaften kann es sinnvoll sein, zusätzliche monoethylenisch ungesättigte Verbindungen einzusetzen, die keine Säuregruppen tragen, aber mit den säuregruppentragenden Monomeren copolymerisierbar sind. Hierzu gehören beispielsweise die Amide und Nitrile von monoethylenisch ungesättigten Carbonsäure, z. B. Acrylamid, Methacrylamid und N-Vinylformamid, N-Vinylacetamid, N-Methyl-vinylacetamid, Acrylnitril und Methacrylnitril. Weitere geeignete Verbindungen sind beispielsweise Vinylester von gesättigten $C_1$- bis $C_4$-Carbonsäuren wie Vinylformiat, Vinylacetat oder Vinylpropionat, Alkylvinylether mit mindestens 2 C-Atomen in der Alkylgruppe, wie z. B. Ethylvinylether oder Butylvinylether, Ester von monoethylenisch ungesättigten $C_3$- bis $C_6$-Carbonsäuren, z. B. Ester aus einwertigen $C_1$- bis $C_{18}$-Alkoholen und Acrylsäure, Methacrylsäure oder Maleinsäure, Halbester von Maleinsäure, z. B.

Maleinsäuremono-methylester, N-Vinyllactame wie N-Vinylpyrrolidon oder N-Vinylcaprolactam, Acrylsäure- und Methacrylsäureester von alkoxylierten einwertigen, gesättigten Alkoholen, z. B. von Alkoholen mit 10 bis 25 C-Atome, die mit 2 bis 200 Mol Ethylenoxid und/oder Propylenoxid pro Mol Alkohol umgesetzt worden sind, sowie Monoacrylsäureester und Monomethacrylsäureester von Polyethylenglykol oder Polypropylenglykol, wobei die Molmassen ($M_n$) der Polyalkylenglykole beispielsweise bis zu 2000 betragen können. Weiterhin geeignete Monomere sind Styrol und alkylsubstituierte Styrole wie Ethylstyrol oder tert.-Butylstyrol.

Diese keine säuregruppentragenden Monomere können auch in Mischung mit anderen Monomeren eingesetzt werden, z. B. Mischungen aus Vinylacetat und 2-Hydroxyethylacrylat in beliebigem Verhältnis. Diese keine Säuregruppen tragenden Monomere werden der Reaktionsmischung in Mengen zwischen 0 und 50 Gew.-%, vorzugsweise kleiner 20 Gew.-% zugesetzt.

Bevorzugt werden vernetzte Polymere aus Säuregruppen tragenden monoethylenisch ungesättigten Monomeren, die gegebenenfalls vor oder nach der Polymerisation in ihre Alkali- oder Ammoniumsalze überführt werden, und aus 0 - 40 Gew.-% bezogen auf ihr Gesamtgewicht keine säuregruppentragenden monoethylenisch ungesättigten Monomeren.

Bevorzugt werden vernetzte Polymere aus monoethylenisch ungesättigten $C_3$- bis $C_{12}$-Carbonsäuren und/oder deren Alkali- oder Ammoniumsalzen. Insbesondere werden vernetzte Polyacrylsäuren bevorzugt, deren Säuregruppen zu 5 bis 80 Mol%, vorzugsweise 60 bis 80 Mol% und bei sauren Superabsorbern 5 - 30 Mol%, vorzugsweise bei 5 bis 20 Mol%, besonders bevorzugt bei 5 bis 10 Mol%, bezogen auf die Säuregruppen enthaltenden Monomere als Alkali- oder Ammoniumsalze vorliegen.

Als Vernetzer können Verbindungen fungieren, die mindestens zwei ethylenisch ungesättigte Doppelbindungen aufweisen. Beispiele für Verbindungen dieses Typs sind N,N'-Methylenbisacrylamid, Polyethylenglykoldiacrylate und Polyethylenglykoldimethacrylate, die sich jeweils von Polyethylenglykolen eines Molekulargewichts von 106 bis 8500, vorzugsweise 400 bis 2000, ableiten, Trimethylolpropantriacrylat, Trimethylolpropantrimethacrylat, Ethylenglykoldiacrylat, Ethylenglykoldimethacrylat, Propylenglykoldiacrylat Propylenglykoldimethacrylat, Butandioldiacrylat, Butandioldimethacrylat, Hexandioldiacrylat, Hexandioldimethacrylat, Allylmethacrylat, Diacrylate und Dimethacrylate von Blockcopolymerisaten aus Ethylenoxid und Propylenoxid, zweifach bzw. mehrfach mit Acrylsäure oder Methacrylsäure veresterte mehrwertige Alkohole, wie Glycerin oder Pentaerythrit, Triallylamin, Dialkyldiallylammoniumhalogenide wie Dimethyldiallylammoniumchlorid und Diethyldiallylammoniumchlorid, Tetraallylethylendiamin, Divinylbenzol, Diallylphthalat, Polyethylenglykoldivinylether von Polyethylenglykolen eines Molekulargewichtes von 106 bis 4000, Trimethylolpropandiallylether, Butandioldivinylether, Pentaerythrittriallylether, Umsetzungsprodukte von 1 Mol Ethylenglykoldiglycidylether oder Polyethylenglykoldiglycidylether mit 2 Mol Pentaerythritoltriallylether oder Allylalkohol, und/oder Divinylethylenharnstoff. Vorzugsweise setzt man wasserlösliche Vernetzer ein, z. B. N,N'-Methylenbisacrylamid, Polyethylenglykoldiacrylate und Polyethylenglykol-dimethacrylate, die sich von Additionsprodukten von 2 bis 400 Mol Ethylenoxid an 1 Mol eines Diols oder Polyols ableiten, Vinylether von Additionsprodukten von 2 bis 400 Mol Ethylenoxid an 1 Mol eines Diols oder Polyols, Ethylenglykoldiacrylat, Ethylenglykoldimethacrylat oder Triacrylate und Trimethacrylate von Additionsprodukten von 6 bis 20 Mol Ethylenoxid an 1 Mol Glycerin, Pentaerythrittriallylether und/oder Divinylharnstoff.

Als Vernetzer kommen außerdem Verbindungen in Betracht, die mindestens eine polymerisierbare ethylenisch ungesättigte Gruppe und mindestens eine weitere funktionelle Gruppe enthalten. Die funktionelle Gruppe dieser Vernetzer muss in der Lage sein, mit den funktionellen Gruppen, im wesentlichen den Säuregruppen, der Monomeren zu reagieren. Geeignete funktionelle Gruppen sind beispielsweise Hydroxyl-, Amino-, Epoxi- und Aziridinogruppen. Verwendung finden können z. B. Hydroxyalkylester der oben genannten monoethylenisch ungesättigten Carbonsäuren, z. B. 2-Hydroxyethylacrylat, Hydroxypropylacrylat, Hydroxybutylacrylat, Hydroxyethylmethacrylat, Hydroxypropylmethacrylat und Hydroxybutylmethacrylat, Allylpiperidiniumbromid, N-Vinylimidazole wie z. B. N-Vinylimidazol, 1-Vinyl-2-methylimidazol und N-Vinylimidazoline wie N-Vinylimidazolin, 1-Vinyl-2-methylimidazolin, 1-Vinyl-2-ethylimidazolin oder 1-Vinyl-2-propylimidazolin, die in Form der freien Basen, in quaternisierter Form oder als Salz bei der Polymerisation eingesetzt werden können. Außerdem eignen sich Dialkylaminoethylacrylat, Dimethylaminoethylmethacrylat, Diethylaminoethylacrylat und Diethylaminoethylmethacrylat. Die basischen Ester werden vorzugsweise in quaternisierter Form oder als Salz eingesetzt. Weiterhin kann z. B. auch Glycidyl(meth)acrylat eingesetzt werden.

Weiterhin kommen als Vernetzer Verbindungen in Betracht, die mindestens zwei funktionelle Gruppen enthalten, die in der Lage sind, mit den funktionellen Gruppen, im wesentlichen den Säuregruppen der Monomeren zu reagieren. Die hierfür geeigneten funktionellen Gruppen wurden bereits oben genannt, d. h. Hydroxyl-, Amino-, Epoxi-, Isocyanat-, Ester-, Amido- und Aziridinogruppen. Beispiele für solche Vernetzer sind Ethylenglykol, Diethylenglykol, Triethylenglykol, Tetraethylenglykol, Polyethylenglykol, Glycerin, Polyglycerin, Triethanolamin, Propylenglykol, Polypropylenglykol, Blockcopolymerisate aus Ethylenoxid und Propylenoxid, Ethanolamin, Sorbitanfettsäureester, ethoxylierte Sorbitanfettsäureester, Trimethylolpropan, Pentaerythrit, 1,3-Butandiol, 1,4-Butandiol, Polyvinylalkohol, Sorbit, Stärke, Polyglycidylether wie Ethylenglykoldiglycidylether, Polyethylenglykol-diglycidylether, Glycerindiglycidylether, Glycerinpolyglycidylether, Diglycerinpolyglycidylether, Polyglycerinpolyglycidylether, Sorbitpolyglycidylether, Pentaerythritpolyglycidylether, Propylenglykoldiglycidylether und Polypropylenglykoldiglycidylether, Polyaziridinverbindungen wie 2,2-Bishydroxymethylbutanol-tris[3-(1-aziridinyl)-propionat], 1,6-Hexamethylendiethylenharnstoff, Diphenylmethan-bis-4,4'-N,N'-diethylenharnstoff, Halogenepoxyverbindungen wie Epichlorhydrin und $\alpha$-Methylepifluorhydrin, Polyisocyanate wie 2,4-Toluylendiisocyanat und Hexamethylendiisocyanat, Alkylencarbonate wie 1,3-Dioxolan-2-on und 4-Methyl-1,3-dioxolan-2-on, weiterhin Bisoxazoline und Oxazolidone, Polyamidoamine sowie deren Umsetzungsprodukte mit Epichlorhydrin, ferner polyquaternäre Amine wie Kondensationsprodukte von Dimethylamin mit Epichlorhydrin, Homo- und Copolymere von Diallyldimethylammoniumchlorid sowie Homo- und Copolymerisate von Dimethylaminoethyl(meth)acrylat, die gegebenenfalls mit beispielsweise Methylchlorid quaterniert sind.

Weitere geeignete Vernetzer sind polyvalente Metallionen, die in der Lage sind, ionische Vernetzungen auszubilden. Beispiele für solche Vernetzer sind Magnesium-, Calcium-, Barium- und Aluminiumionen. Diese Vernetzer werden beispielsweise als Hydroxide, Carbonate oder Hydrogencarbonate eingesetzt. Weitere geeignete Vernetzer sind multifunktionelle Basen, die ebenfalls in der Lage sind, ionische Vernetzungen auszubilden, beispielsweise Polyamine oder deren quaternierte Salze. Beispiele für Polyamine sind Ethylendiamin, Diethylentriamin, Triethylentetramin, Tetraethylenpentamin, Pentaethylenhexamin und Polyethylenimine sowie Polyamine mit Molmassen von jeweils bis zu 4000000. Die Vernetzer sind in der Reaktionsmischung beispielsweise von 0,001 bis 20 und vorzugsweise von 0,01 bis 14 Gew.-% vorhanden.

b) Radikalische Polymerisation

Die Polymerisation wird wie allgemein üblich durch einen Initiator ausgelöst. Auch eine Initiierung der Polymerisation durch Einwirkung von Elektronenstrahlen auf die polymerisierbare, wässrige Mischung ist möglich. Die Polymerisation kann allerdings auch in Abwesenheit von Initiatoren der obengenannten Art durch Einwirkung energiereicher Strahlung in Gegenwart von Photoinitiatoren ausgelöst werden. Als Polymerisationsinitiatoren können sämtliche unter den Polymerisationsbedingungen in Radikale zerfallende Verbindungen eingesetzt werden, z. B. Peroxide, Hydroperoxide, Wasserstoffperoxide, Persulfate, Azoverbindungen und die sogenannten Redoxkatalysatoren. Bevorzugt ist der Einsatz von wasserlöslichen Initiatoren. In manchen Fällen ist es vorteilhaft, Mischungen verschiedener Polymerisationsinitiatoren zu verwenden, z. B. Mischungen aus Wasserstoffperoxid und Natrium- oder Kaliumperoxodisulfat. Mischungen aus Wasserstoffperoxid und Natriumperoxodisulfat können in jedem beliebigen Verhältnis verwendet werden. Geeignete organische Peroxide sind beispielsweise Acetylacetonperoxid, Methylethylketonperoxid, tert.-Butylhydroperoxid, Cumolhydroperoxid, tert.-Amylperpivalat, tert.-Butylperpivalat, tert.-Butylperneohexanoat, tert.-Butylperisobutyrat, tert.-Butyl-per-2-ethylhexanoat, tert.-Butylperisononanoat, tert.-Butylpermaleat, tert.-Butylperbenzoat, Di-(2-ethylhexyl)peroxidicarbonat, Dicyclohexylperoxidicarbonat, Di-(4-tert.-butylcyclohexyl)peroxidicarbonat, Dimyristilperoxidicarbonat, Diacetylperoxi-dicarbonat, Allylperester, Cumylperoxyneodecanoat, tert.-Butylper-3,5,5-trimethylhexanoat, Acetylcyclohexylsulfonylperoxid, Dilaurylperoxid, Dibenzoylperoxid und tert.-Amylperneodekanoat. Besonders geeignete Polymerisationsinitiatoren sind wasserlösliche Azostarter, z. B. 2,2'-Azo-bis-(2-amidinopropan)dihydrochlorid, 2,2'-Azo-bis-(N,N'-dimethylen)isobutyramidin-dihydrochlorid, 2-(Carbamoylazo)isobutyronitril, 2,2'-Azo-bis[2-(2'-imidazolin-2-yl)propan]dihydrochlorid und 4,4'-Azo-bis-(4-cyanovaleriansäure). Die genannten Polymerisationsinitiatoren werden in üblichen Mengen eingesetzt, z. B. in Mengen von 0,01 bis 5, vorzugsweise 0,05 bis 2,0 Gew.-%, bezogen auf die zu polymerisierenden Monomeren.

Als Initiatoren kommen außerdem Redoxkatalysatoren in Betracht. Die Redoxkatalysatoren enthalten als oxidierende Komponente mindestens eine der oben angegebenen Perverbindungen und als reduzierende Komponente beispielsweise Ascorbinsäure, Glukose, Sorbose, Ammonium- oder Alkalimetallhydrogensulfit, -sulfit, -thiosulfat, -hyposulfit, -pyro-sulfit oder -sulfid, Metallsalze, wie Eisen(II)-ionen oder Natriumhydroxymethylsulfoxylat. Vorzugsweise verwendet man als reduzierende Komponente des Redoxkatalysators Ascorbinsäure oder Natriumsulfit. Bezogen auf die bei der Polymerisation eingesetzte Menge an Monomeren verwendet man beispielsweise 3 x $10^{-6}$ bis 1 Mol-% der reduzierenden Komponente des Redoxkatalysatorsystems und 0,001 bis 5,0 Mol-% der oxidierenden Komponente des Redoxkatalysators.

Wenn man die Polymerisation durch Einwirkung energiereicher Strahlung auslöst, verwendet man üblicherweise als Initiator sogenannte Photoinitiatoren. Hierbei kann es sich beispielsweise um sogenannte $\alpha$-Spalter, H-abstrahierende Systeme oder auch um Azide handeln. Beispiele für solche Initiatoren sind Benzophenon-Derivate wie Michlers-Keton, Phenanthren-Derivate, Fluoren-Derivate, Anthrachinon-Derivate, Thioxanton-Derivate, Cumarin-Derivate, Benzoinether und deren Derivate, Azoverbindungen wie die oben genannten Radikalbildner, substituierte Hexaarylbisimidazole oder Acylphosphinoxide. Beispiele für Azide sind: 2-(N,N-Dimethylamino)-ethyl-4-azidocinnamat, 2-(N,N-Dimethyl-amino)-ethyl-4-azidonaphthylketon, 2-(N,N-Dimethylamino)-ethyl-4-azidobenzoat, 5-Azido-1-naphthyl-2'-(N,N-dimethylamino)ethylsulfon, N-(4-Sulfonylazidophenyl)maleinimid, N-Acetyl-4-sulfonylazidoanilin, 4-Sulfonylazidoanilin, 4-Azidoanilin, 4-Azidophenacylbromid, p-Azidobenzoesäure, 2,6-Bis(p-azidobenzyliden)cyclohexanon und 2,6-Bis-(p-azido-benzyliden)-4-methylcyclohexanon. Die Photoinitiatoren werden, falls sie eingesetzt werden, üblicherweise in Mengen von 0,01 bis 5 Gew.-% bezogen auf die zu polymerisierenden Monomeren angewendet.

Die vernetzten Polymere werden vorzugsweise teilneutralisiert eingesetzt. Der Neutralisationsgrad beträgt 5 - 80 Mol%, bei neutralen Superabsorbern bevorzugt 60 - 80 Mol%, bei sauren Superabsorbern bevorzugt 5 bis 60 Mol%, vorzugsweise 10 bis 40 Mol%, besonders bevorzugt 20 bis 30 Mol%, bezogen auf die Säuregruppen enthaltenden Monomere. Als Neutralisationsmittel kommen in Frage: Alkalimetallbasen oder Ammoniak bzw. Amine. Vorzugsweise wird Natronlauge, Kalilauge oder Lithiumhydroxid verwendet. Die Neutralisation kann jedoch auch mit Hilfe von Natriumcarbonat, Natriumhydrogencarbonat, Kaliumcarbonat oder Kaliumhydrogencarbonat oder anderen Carbonaten oder Hydrogencarbonaten oder Ammoniak vorgenommen werden. Darüberhinaus sind prim., sek. und tert. Amine einsetzbar.

Alternativ kann die Einstellung des Neutralisationsgrades vor, während oder nach der Polymerisation in allen dafür geeigneten Apparaturen vorgenommen werden. Die Neutralisation kann beispielsweise bei Verwendung eines Kneters zur Polymerisation auch direkt hierin vorgenommen werden.

Als technische Verfahren zur Herstellung dieser Produkte können alle Verfahren Anwendung finden, die üblicherweise bei der Herstellung von Superabsorbern eingesetzt werden, wie sie z. B. im Kapitel 3 in "Modern Superabsorbent Polymer Technology", F.L. Buchholz and A.T. Graham, Wiley-VCH, 1998, erläutert sind.

Bevorzugt ist die Polymerisation in wässriger Lösung als sogenannte Gelpolymerisation. Dabei werden 10 bis 70 Gew.-%ige wässrige Lösungen der Monomere und gegebenenfalls einer geeigneten Pfropfgrundlage in Gegenwart eines Radikalinitiators unter Ausnutzung des Tromansdorff-Norrish-Effektes polymerisiert.

Die Polymerisationsreaktion kann im Temperaturbereich zwischen 0 °C und 150 °C, vorzugsweise zwischen 10 °C und 100 °C, sowohl bei Normaldruck als auch unter erhöhtem oder erniedrigtem Druck durchgeführt werden. Wie üblich kann die Polymerisation auch in einer Schutzgasatmosphäre, vorzugsweise unter Stickstoff, ausgeführt werden.

Durch Nachheizen der Polymerisatgele im Temperaturbereich 50 bis 130 °C, vorzugsweise 70 bis 100 °C, können die Qualitätseigenschaften der Polymerisate noch verbessert werden.

Die bei der Herstellung von Superabsorbern eingesetzte Acrylsäure ist in der Regel mit phenolischen Verbindungen, vorzugsweise p-Methoxyphenol, stabilisiert (Modern Superabsorbent Polymer Technology, John Wiley & sons, Inc., 1998, Kap. 2.2.2.1 u. Kap. 2.5.3). Von der radikalischen Polymerisation muß der Inhibitor unschädlich gemacht werden. Dies geschieht allgemein durch Inertisierung mit Stickstoff oder Kohlendioxid. p-Methoxyphenol benötigt bekanntlich zur wirkungsvollen Stabilisierung Sauerstoff. Nach Beendigung der Polymerisation verbleibt der Inhibitor jedoch im Produkt.

Die Acrylsäure, die zur Herstellung der Absorberharze eingesetzt wird, hat in der Regel folgende Zusammensetzung:

| | |
|---|---|
| Acrylsäure | 99.5 - 99.95 Gew.-% |
| Essigsäure | 0.01 - 0.5 Gew.-% |
| Propionsäure | 0.001 - 0.1 Gew.-% |
| Diacrylsäure | 0.005 - 0.2 Gew.-% |
| Aldehyde | max. 5 ppm |
| Inhibitor (z.B. p-Methoxyphenol) | 150 - 250 ppm |

Für andere säuregruppentragende Monomere gelten entsprechende Konzentrationen. Die Acrylsäure kann nach beliebigen Methoden hergestellt werden.

c) Oberflächennachvernetzung

Bevorzugt werden Hydrogel-formende Polymere, die oberflächennachvernetzt sind. Die Oberflächennachvernetzung kann in an sich bekannter Weise mit getrockneten, gemahlenen und abgesiebten Polymerpartikeln geschehen.
Hierzu werden Verbindungen, die mit den funktionellen Gruppen der Polymere unter Vernetzung reagieren können, vorzugsweise in Form einer wasserhaltigen Lösung auf die Oberfläche der Hydrogel-Partikel aufgebracht. Die wasserhaltige Lösung kann wassermischbare organische Lösungsmittel enthalten. Geeignete Lösungsmittel sind Alkohole wie Methanol, Ethanol, i-Propanol, Ethylenglycol, Propylenglycol oder Aceton.
Bei der nachträglichen Vernetzung werden Polymere, die durch die Polymerisation der oben genannten monoethylenisch ungesättigten Säuren und gegebenenfalls monoethylenisch ungesättigten Comonomere hergestellt wurden und die ein Molekulargewicht größer 5000, bevorzugt größer 50000 aufweisen, mit Verbindungen umgesetzt, die mindestens zwei gegenüber Säuregruppen reaktive Gruppen aufweisen. Diese Umsetzung kann bei Raumtemperatur oder aber bei erhöhten Temperaturen bis zu 220 °C erfolgen.
Geeignete Nachvernetzungsmittel sind beispielsweise

- Di- oder Polyglycidylverbindungen wie Phosphonsäurediglycidylether oder Ethylenglykoldiglycidylether, Bischlorhydrinether von Polyalkylenglykolen,
- Alkoxysilylverbindungen,
- Polyaziridine, Aziridin-Einheiten enthaltende Verbindungen auf Basis von Polyethern oder substituierten Kohlenwasserstoffen, beispielsweise Bis-N-aziridinomethan,
- Polyamine oder Polyamidoamine sowie deren Umsetzungsprodukte mit Epichlorhydrin,
- Polyole wie Ethylenglykol, 1,2-Propandiol, 1,4-Butandiol, Glycerin, Methyltriglykol, Polyethylenglykole mit einem mittleren Molekulargewicht $M_w$ von 200 - 10000, Di- und Polyglycerin, Pentaerythrit, Sorbit, die Oxethylate dieser Polyole sowie deren Ester mit Carbonsäuren oder der Kohlensäure wie Ethylencarbonat oder Propylencarbonat,
- Kohlensäurederivate wie Harnstoff, Thioharnstoff, Guanidin, Dicyandiamid, 2-Oxazolidinon und dessen Derivate, Bisoxazolin, Polyoxazoline, Di- und Polyisocyanate,
- Di- und Poly-N-methylolverbindungen wie beispielsweise Methylenbis(N-methylol-methacrylamid) oder Melamin-Formaldehyd-Harze,
- Verbindungen mit zwei oder mehr blockierten Isocyanat-Gruppen wie beispielsweise Trimethylhexamethylendiisocyanat blockiert mit 2.,2,3,6-Tetramethyl-piperidinon-4.

Bei Bedarf können saure Katalysatoren wie beispielsweise p-Toluolsulfonsäure, Phosphorsäure, Borsäure oder Ammoniumdihydrogenphosphat zugesetzt werden.
Besonders geeignete Nachvernetzungsmittel sind Di- oder Polyglycidylverbindungen wie Ethylenglykoldiglycidylether, die Umsetzungsprodukte von Polyamidoaminen mit Epichlorhydrin und 2-Oxazolidinon.
Das Aufbringen der Vernetzer-Lösung erfolgt bevorzugt durch Aufsprühen einer Lösung des Vernetzers in herkömmlichen Reaktionsmischern oder Misch- und Trocknungsanlagen wie beispielsweise Patterson-Kelly-Mischer, DRAIS-Turbulenzmischer, Lödige-Mischer, Schneckenmischer, Tellermischer, Wirbelschichtmischer und Schugi-Mix. Nach Aufsprühen der Vernetzer-Lösung kann ein Temperaturbehandlungsschritt nachfolgen, bevorzugt in einem nachgeschalteten Trockner, bei einer Temperatur zwischen 80 und 230 °C, bevorzugt 80 - 190 °C, und besonders bevorzugt zwischen 100 und 160 °C, über einen Zeitraum von 5 Minuten bis 6 Stunden, bevorzugt 10 Minuten bis 2 Stunden und besonders bevorzugt 10 Minuten bis 1 Stunde, wobei sowohl Spaltprodukte als auch Lösungsmittelanteile entfernt werden können. Die Trocknung kann aber auch im Mischer selbst erfolgen, durch Beheizung des Mantels oder Einblasen eines vorgewärmten Trägergases.
In einer besonders bevorzugten Ausführung der Erfindung wird zusätzlich die Hydrophilie der Partikeloberfläche der Hydrogel-formenden Polymere durch Ausbildung von Komplexen modifiziert. Die Bildung der Komplexe auf der äußeren Schale der Hydrogel-Partikel erfolgt durch Aufsprühen von Lösungen zwei- oder mehrwertiger Metallsalz-Lösungen, wobei die Metall-Kationen mit den Säuregruppen des Polymers unter Ausbildung von Komplexen reagieren können. Beispiele für zwei- oder mehrwertige Metall-Kationen sind $Mg^{2+}$, $Ca^{2+}$, $Al^{3+}$, $Sc^{3+}$, $Ti^{4+}$, $Mn^{2+}$, $Fe^{2+/3+}$, $Co^{2+}$, $Ni^{2+}$, $Cu^{+/2+}$, $Zn^{2+}$, $Y^{3+}$, $Zr^{4+}$, $Ag^{+}$, $La^{3+}$, $Ce^{4+}$, $Hf^{4+}$, und $Au^{+/3+}$, bevorzugte Metall-Kationen sind $Mg^{2+}$, $Ca^{2+}$, $Al^{3+}$, $Ti^{4+}$, $Zr^{4+}$ und $La^{3+}$, und besonders bevorzugte Metall-Kationen sind $Al^{3+}$, $Ti^{4+}$und $Zr^{4+}$. Die Metall-Kationen können sowohl allein als auch im Gemisch untereinander eingesetzt werden. Von den genannten Metall-Kationen sind alle Metallsalze geeignet, die eine ausreichende Löslichkeit in dem zu verwendenden Lösungsmittel besitzen. Besonders geeignet sind Metallsalze mit schwach komplexierenden Anionen wie zum Beispiel Chlorid, Nitrat und Sulfat. Als Lösungsmittel für die Metallsalze können Wasser, Alkohole, DMF, DMSO sowie Mischungen dieser Komponenten eingesetzt werden.

Besonders bevorzugt sind Wasser und Wasser/Alkohol-Mischungen wie zum Beispiel Wasser/Methanol oder Wasser/1,2-Propandiol.

Das Aufsprühen der Metallsalz-Lösung auf die Partikel des Hydrogel-formenden Polymers kann sowohl vor als auch nach der Oberflächennachverrietzung der Partikel erfolgen. In einem besonders bevorzugten Verfahren erfolgt die Aufsprühung der Metallsalz-Lösung im gleichen Schritt mit dem Aufsprühen der Vernetzer-Lösung, wobei beide Lösungen getrennt nacheinander oder gleichzeitig über zwei Düsen aufgesprüht werden, oder Vernetzer- und Metallsalz-Lösung vereint über eine Düse aufgesprüht werden können.

Optional kann noch eine weitere Modifizierung der Hydrogel-formenden Polymere durch Zumischung feinteiliger anorganischer Feststoffe, wie zum Beispiel Silica, Aluminiumoxid, Titandioxid und Eisen(II)-oxid erfolgen, wodurch die Effekte der Oberflächennachbehandlung noch weiter verstärkt werden. Besonders bevorzugt ist die Zumischung von hydrophilem Silica oder von Aluminiumoxid mit einer mittleren Größe der Primärteilchen von 4 bis 50 nm und einer spezifischen Oberfläche von 50 - 450 $m^2/g$. Die Zumischung feinteiliger anorganischer Feststoffe erfolgt bevorzugt nach der Oberflächenmodifizierung durch Vernetzung/Komplexbildung, kann aber auch vor oder während diesen Oberflächenmodifizierungen durchgeführt werden.

Eigenschaften der erfindungsgemäßen Hydrogel-formender Polymere, des Verfahren zur Herstellung und der verwendeten Acrylsäure.

Die erfindungsgemäßen wässrige Flüssigkeiten absorbierenden Hydrogel-formenden Polymere weisen bei hohem Endabsorptionsvermögen hohe Gelfestigkeit und Permeabilität, sowie hohe Retention auf.

Bevorzugt liegt der SFC-Wert [in $10^{-7}$ $cm^3s/g$] der erfindungsgemäßen Hydrogel-formenden Polymere, der nach den in der Beschreibung angegebenen Methoden gemessen werden kann, bei größer 1, insbesondere 2, 4, 6, 8, 10, 12, 14, 16, 18, 20 oder höher, besonders bevorzugt bei 22 insbesondere bei 24, 26, 28, 30, 32 oder höher.

Bevorzugt liegt der CRC-Wert [g/g] der erfindungsgemäßen Hydrogel-formenden Polymere, der nach den in der Beschreibung angegebenen Methoden gemessen werden kann, bei größer 15, insbesondere 16, 18, 20, 22, 24, oder höher, besonders bevorzugt bei 25, insbesondere bei 26, 27, 28, 29, 30, 31, 32 oder höher.

Bevorzugt liegt der AUL-0.7psi-Wert [g/g] der erfindungsgemäßen Hydrogel-formenden Polymere, der nach den in der Beschreibung angegebenen Methoden gemessen werden kann, bei größer 4, insbesondere 6, 8, 10, 12, oder höher, besonders bevorzugt bei 13 insbesondere bei 14, 15, 16, 17, 18, 19, 20, 21, 22, 23, 24, 25, 26, 27, 28, oder höher.

Besonders bevorzugt ist eine Kombination der Schwellenwerte, wie z.B. SFC mit CRC, SFC mit AUL, AUL mit CRC, insbesondere Dreier Kombinationen SFC, AUL und CRC.

Unter Polymeren auf Basis von Polyacrylat werden erfindungsgemäß solche wässrige Flüssigkeiten absorbierenden Hydrogel-formenden Polymere verstanden, die zumindest Polyacrylate enthalten. Bei Pfropfpolymeren ist bevorzugt Polyacrylat aufgepfropft. Der Anteil an Acrylsäure als hydrophiles Monomer bei (Co)-polymerisierten Polymeren oder bei Pfropf-(co)-polymeren beträgt bevorzugt 50 Gew% oder mehr, bevorzugt 80 Gew% oder mehr, besonders bevorzugt mehr als 90 Gew%, 95 Gew%, 98 Gew%, insbesondere 99 Gew% und mehr.

Unter Tocopherol werden Verbindungen der Folgenden Formel verstanden

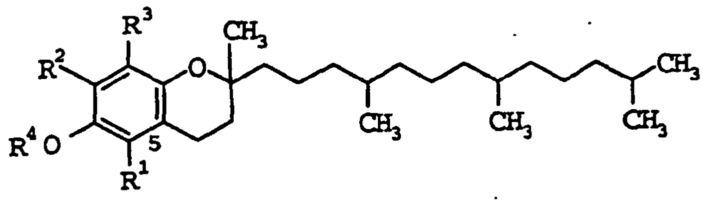

wobei $R^1$ H oder Methyl $R^2$ H oder Methyl $R^3$ H oder Methyl und
$R^4$ H oder Säurerest mit 1 - 20 Kohlenstoffatomen bedeutet.

Bevorzugte Reste für $R^4$ sind Acetyl, Ascorbyl, Succinyl, Nicotinyl und andere physiologisch verträgliche Carbonsäuren. Die Carbonsäuren können Mono-, Di- oder Tricarbonsäuren sein.

Bevorzugt ist alpha-Tocopherol mit R1 = R2 = R3 =Methyl, insbesondere racemisches $\alpha$-Tocopherol. R4 ist besonders bevorzugt H oder Acetyl. Insbesondere bevorzugt ist RRR-alpha-Tocopherol.

Tocopherol liegt im Polymeren und in der nicht polymerisierten Acrylsäure bevorzugt in einer Konzentration von 10 - 1000 ppm, bevorzugt bei 50 - 500 ppm, insbesondere bei 100 - 300 ppm bezogen auf säuregruppentragende Monomere, insbesondere auf Acrylsäure, beziehungsweise Säureeinheiten im Polymeren vor.

Bevorzugt ist säuregruppentragendes Monomer, insbesondere Acrylsäure, das im wesentlichen nur Tocopherol als Stabilisator enthält. Unter im wesentlichen wird verstanden, daß Tocopherol bezüglich der mol-% der Stabilisatoren, den höchsten Anteil hat, bevorzugt mehr als 90 mol%, insbesondere mehr als 95, 96, 97, 98, 99 mol%. Bei der radikalischen Polymerisation hat α-Tocopherol gegenüber p-Methoxyphenol den Vorteil, dass die radikalische Polymerisation rascher startet und besser abläuft. Die erhaltenen Produkte sind weniger gelblich und besitzen einen Z%-Wert von größer 70, insbesondere größer 75 gemessen mit einem Hunterlab LS 5100 Colorimeter. Die Induktionsperiode bei dem erfinderischen Verfahren z.B. mit Acrylsäure ist ≤ 20 sec, insbesondere ≤ 15 sec und damit deutlich kürzer als beim Zusatz von MEHQ.

EP 449 913 (US 5 159 106) beschreibt ein Verfahren zur Herstellung von (Meth)acrylsäureestern mehrwertiger Alkanole durch eine säurekatalysierte Veresterungsreaktion in Gegenwart von Tocopherolen. Eine Verfärbung der Veresterungsprodukte soll dadurch verhindert werden.

WO 99/01410 (EP 998 437) empfiehlt die Verwendung von alpha-Tocopherol als Polymerisationsinhibitors bei der Herstellung, Lagerung und beim Transport von Vinylmonomeren, vorzugsweise Acrylnitril.

US 5,461,124 beschreibt die Verwendung von Tocopherol bei der Herstellung von chirugischen Klebern und chirugischem Zement.

Einsatz und Verwendung Hydrogel-formender Polymerer

Die vorliegende Erfindung betrifft ferner die Verwendung der oben genannten Hydrogel-formenden Polymeren in Hygieneartikel, umfassend

(A) eine obere flüssigkeitsdurchlässige Abdeckung
(B) eine untere flüssigkeitsundurchlässige Schicht
(C) einen zwischen (A) und (B) befindlichen Kern, enthaltend

  (C1) 10 - 100 Gew.-% des erfindungsgemäßem Hydrogel-formenden Polymer
  (C2) 0 - 90 Gew.-% hydrophiles Fasermaterial

(D) gegebenenfalls eine sich unmittelbar oberhalb und unterhalb des Kerns (C) sich befindende Tissue-schicht und
(E) gegebenenfalls eine zwischen (A) und (C) sich befindende Aufnahmeschicht.

Unter Hygieneartikel sind dabei sowohl Inkontinenzeinlagen und Inkontinenzhosen für Erwachsene als auch Windeln für Babys zu verstehen.

Bei der flüssigkeitsdurchlässigen Abdeckung (A) handelt es sich um die Schicht, die direkten Hautkontakt hat. Das Material hierfür besteht hierbei aus üblichen synthetischen oder halbsynthetischen Fasern oder Filme von Polyester, Polyolefine, Rayon oder natürlichen Fasern wie Baumwolle. Bei nichtgewebten Materialien sind die Fasern in der Regel durch Bindemittel wie Polyacrylate zu verbinden. Bevorzugte Materialien sind Polyester, Rayon und deren Blends, Polyethylen und Polypropylen.

Die flüssigkeitsundurchlässige Schicht (B) besteht in der Regel aus einer Folie aus Polyethylen oder Polypropylen.

Der Kern (C) enthält neben dem erfindungsgemäßen Hydrogel-formendem Polymer (C1) hydrophiles Fasermaterial (C2). Unter hydrophil ist zu verstehen, dass sich wässrige Flüssigkeiten schnell über die Faser verteilen. Für gewöhnlich ist das Fasermaterial Cellulose, modifizierte Cellulose, Rayon, Polyester wie Polyethylenterephthalat. Besonders bevorzugt werden Cellulosefasern wie Zellstoff. Die Fasern haben in der Regel einen Durchmesser von 1 - 200 μm, bevorzugt 10 - 100 μm. Darüber hinaus haben die Fasern eine Mindestlänge von 1 mm.

Der Anteil des hydrophilen Fasermaterials bezogen auf die Gesamtmenge des Kerns beträgt bevorzugt 20-80 Gew.-%, besonders bevorzugt 40 - 70 Gew.-%.

Der Aufbau und die Form von Windeln ist allgemein bekannt und beispielsweise in der EP-A-0 316 518 und EP-A-0 202 127 beschrieben. Allgemein werden Windeln und andere Hygieneartikel auch in WO 00/65084, insbesondere auf Seiten 6-15, WO 00/65348, insbesondere auf Seiten 4 - 17, WO 00/35502, insbesondere Seiten 3-9, DE 19737434, WO 98/8439 beschrieben.

Die erfindungsgemäßen sauren Hydrogel-formenden Polymere eignen sich hervorragend als Absorptionsmittel für Wasser und wässrige Flüssigkeiten, so dass sie vorteilhaft als Wasser zurückhaltendes Mittel im landwirtschaftlichen Gartenbau, als Filtrationshilfsmittel und besonders als saugfähige Komponente in Hygieneartikeln wie Windeln, Tampons oder Damenbinden eingesetzt werden können.

Experimenteller Teil

Testmethoden

a) Zentrifugenretentionskapazität (CRC Centrifuge Retention Capacity)

Bei dieser Methode wird die freie Quellbarkeit des Hydrogels im Teebeutel bestimmt. Zur Bestimmung der CRC werden 0.2000 ± 0.0050 g getrocknetes Hydrogel (Kornfraktion 106 - 850 μm) in einem 60 x 85 mm großen Teebeutel eingewogen, der anschließend verschweißt wird. Der Teebeutel wird für 30 Minuten in einen Überschuss von 0.9 Gew.%igen Kochsalzlösung gegeben (mindestens 0,83 l KochsalzLösung / 1 g Polymerpulver). Anschließend wird der Teebeutel 3 Minuten lang bei 250 g zentrifugiert. Die Bestimmung der Flüssigkeitsmenge geschieht durch Auswägen des zentrifugierten Teebeutels.

b) Absorption unter Druck (AUL Absorbency Under Load) (0.7 psi)

Die Messzelle zur Bestimmung der AUL 0.7 psi stellt ein Plexiglas-Zylinder mit einem Innendurchmesser von 60 mm und einer Höhe von 50 mm dar, der an der Unterseite einen angeklebten Edelstahl-Siebboden mit einer Maschenweite von 36 μm besitzt. Zu der Messzelle gehört weiterhin eine Plastikplatte mit einem Durchmesser von 59 mm und ein Gewicht, welches zusammen mit der Plastikplatte in die Messzelle hineingestellt werden kann. Das Gewicht der Plastikplatte und das Gewicht betragen zusammen 1345 g. Zur Durchführung der Bestimmung der AUL 0.7 psi wird das Gewicht des leeren Plexiglas-Zylinders und der Plastikplatte ermittelt und als $W_0$ notiert. Dann werden 0,900 ± 0.005 g Hydrogel-formendes Polymer (Korngrößenverteilung 150 - 800 μm) in den Plexiglas-Zylinder eingewogen und möglichst gleichmäßig auf dem Edelstahl-Siebboden verteilt. Anschließend wird die Plastikplatte vorsichtig in den Plexiglas-Zylinder hineingelegt und die gesamte Einheit gewogen; das Gewicht wird als $W_a$ notiert. Nun wird das Gewicht auf die Plastikplatte in dem Plexiglas-Zylinder gestellt. In die Mitte der Petrischale mit einem Durchmesser von 200 mm und einer Höhe von 30 mm wird eine keramische Filterplatte mit einem Durchmesser von 120 mm und einer Porosität 0 gelegt und soviel 0.9 Gew.%ige Natriumchloridlösung eingefüllt, dass die Flüssigkeitsoberfläche mit der Filterplattenoberfläche abschließt, ohne dass die Oberfläche der Filterplatte benetzt wird. Anschließend wird ein rundes Filterpapier mit einem Durchmesser von 90 mm und einer Porengröße < 20 μm (S&S 589 Schwarzband von Schleicher & Schüll) auf die keramische Platte gelegt. Der Hydrogel-formendes Polymer enthaltende Plexiglas-Zylinder wird mit Plastikplatte und Gewicht nun auf das Filterpapier gestellt und dort für 60 Minuten belassen. Nach dieser Zeit wird die komplette Einheit aus der Petrischale vom Filterpapier herausgenommen und anschließend das Gewicht aus dem Plexiglas-Zylinder entfernt. Der gequollenes Hydrogel enthaltende Plexiglas-Zylinder wird zusammen mit der Plastikplatte ausgewogen und das Gewicht als $W_b$ notiert.

Die Absorption unter Druck (AUL) wird wie folgt berechnet:

$$\text{AUL 0.7 psi [g/g]} = [W_b - W_a] \,/\, [W_a - W_0]$$

c) Saline Flow, Conductivity (SFC)

Die Testmethode zur Bestimmung der SFC ist beschrieben in U.S. 5 599 335.

Beispiele

Beispiel SAP-Herstellung

Ausgehend von 1735 g Acrylsäure, versehen mit racemischen α-Tocopherol bzw. MEHQ, 1445 g 50%-iger Natronlauge und 2760 g Wasser wurde auf übliche Weise eine ca. 30 %-ige Natriumacrylatlösung hergestellt und auf übliche Weise mittels einer Strippkolonne im Gegenstrom mit Stickstoff entoxigeniert.

Die weitgehend sauerstofffreie Lösung wurde in einen Trogkneter (Typ LUK 8, Werner & Pfleiderer) überführt und mit 7,8 g Polyethylenglykoldiacrylat versetzt und gut durchmischt. Der Reaktor wurde während der ganzen Reaktionszeit mit Stickstoff überlagert.

Bei eingeschalteten Rührwellen wurde das Startersystem, zuerst 32,12 g Natriumpersulfat (15 %-ige Lösung) und dann 20,79 g Ascorbinsäure (0,5 %-ig), zugegeben. Nach Beendigung der Zugabe wurde bei einer Heizflüssigkeitstemperatur von 74°C der Inhalt des Kneters erhitzt. Das Gemisch begann sich zu erwärmen und wurde zähviskos (Induktionsperiode) . Sobald die maximale Polymerisationstemperatur überschritten wurde, wurde die Heizung abgeschaltet und ca. 15 Minuten nachpolymerisiert. Der Inhalt des Kneters wurde auf 50 - 60°C abgekühlt und auf einem Trocknungssieb in einer dünnen Schicht aufgebracht und ca. 90 Minuten bei 160°C im Trockenschrank getrocknet. Das getrocknete Pulver wurde anschließend durch mahlen und sieben auf eine Endkorngröße von 100 bis 850 μm gebracht.

Oberflächenvernetzung:

In einem Lödige-Pflugscharmischer von 5 l Inhalt werden 1,8 kg Superabsorber-Pulver, hergestellt gemäß obiger Vorschrift, vorgelegt. Im Verlauf von 5 bis 10 Min. wird eine Lösung von 1,4 g Etyhlenglykoldiglycidylether, 59 g Wasser und 29 g 1,2-Propandiol aufgesprüht. Es wird auf 120°C Produkttemperatur angeheizt und 60 Minuten bei dieser Temperatur gehalten, um das Lösungsmittel wieder abzudestillieren. Anschließend wird abgekühlt, das Produkt ausgetragen und die Kornfraktion 100 - 850 μm abgesiebt.

1. Beispiel

AS mit 250 ppm racemischem α-Tocopherol
Induktionsperiode ≤ 10 sec.
Endprodukt sehr weiß (Z%-Wert: 76)*)

2. Beispiel

AS mit 220 ppm MEHQ
Induktionsperiode 70 sec.
Endprodukt leicht gelb bis gelb (Z%-Wert: 63)*)
Induktionsperiode - α-Tocopherol: typischerweise 5 - 15 sec.
MEHQ: typischerweise 50 - 150 sec.
*) Farbbestimmung (Z%-Werte) mit dem
Hunterlab LS 5100 Colorimeter

## Patentansprüche

1. Wässrige Flüssigkeiten absorbierende Hydrogel-formende Polymere auf Basis von säuregruppentragenden Polymeren, die Tocopherol enthalten.

2. Wässrige Flüssigkeiten absorbierende Hydrogel-formende Polymere gemäß Anspruch 1, wobei das säuregruppentragende Polymer Polyacrylat ist.

3. Wässrige Flüssigkeiten absorbierende Hydrogel-formende Polymere gemäß einem der Ansprüche 1 oder 2, wobei das Tocopherol in dem Polymer verteilt vorliegt.

4. Wässrige Flüssigkeiten absorbierende Hydrogel-formende Polymere gemäß einem der Ansprüche 1 bis 3, wobei das Tocopherol alpha-Tocopherol ist.

5. Wässrige Flüssigkeiten absorbierende Hydrogel-formende Polymere gemäß einem der Ansprüche 1 bis 4, wobei das Tocopherol zwischen 10 und 1000 ppm, bezogen auf säuregruppentragendes Monomer, vorliegt.

6. Verfahren zur Herstellung von wässrige Flüssigkeiten absorbierenden Hydrogel-formenden Polymeren auf Basis von säuregruppentragenden Polymeren, wobei die zur Herstellung verwendeten säuregruppentragenden Monomere Tocopherol enthalten.

7. Verfahren gemäß Anspruch 6, wobei die verwendete Acrylsäure mindestens 50 ppm Tocopherol enthält und die Induktionsperiode höchstens 20 Sekunden beträgt.

8. Verfahren gemäß einem der Ansprüche 6 oder 7, wobei die verwendete Acrylsäure mindestens 50 ppm Tocopherol enthält und der Z%-Wert des erhaltenen wässrige Flüssigkeiten absorbierenden Bydrogel-formenden Polymeren größer 70 ist.

9. Verwendung säuregruppentragender Monomere zur Herstellung wässriger Flüssigkeiten absorbierender Hydrogel-formender Polymere, wobei das säuregruppentragende Monomer Tocopherol, insbesondere alpha-Tocopherol, enthält.

**10.** Verwendung säuregruppentragender Monomere nach Anspruch 9, wobei das säuregruppentragende Monomer nur Tocopherol als Stabilisator enthält.

**11.** Verwendung von Acrylsäure oder Acrylat als säuregruppentragendes Monomer gemäß einem der Ansprüche 9 oder 10.

**12.** Verwendung von wässrigen Flüssigkeiten absorbierenden Hydrogel-formenden Polymeren erhältlich nach einem Verfahren gemäß einem der Ansprüche 6 bis 8 zur Absorption von wässrigen Flüssigkeiten, insbesondere in Hygieneartikeln.

## Claims

**1.** Hydrogel-forming polymers capable of absorbing aqueous fluids and based on acid-functional polymer comprising tocopherol.

**2.** Hydrogel-forming polymers according to claim 1, wherein said acid-functional polymer is polyacrylate.

**3.** Hydrogel-forming polymers according to either of claims 1 and 2, wherein said tocopherol is distributed over the polymer.

**4.** Hydrogel-forming polymers according to any of claims 1 to 3, wherein said tocopherol is alpha-tocopherol.

**5.** Hydrogel-forming polymers according to any of claims 1 to 4, wherein said tocopherol is present in an amount from 10 to 1000 ppm, based on acid-functional monomer.

**6.** A process for preparing hydrogel-forming polymers capable of absorbing aqueous fluids and based on acid-functional polymer, which comprises using acid-functional monomer that comprises tocopherol.

**7.** The process according to claim 6, wherein the acrylic acid used comprises at least 50 ppm of tocopherol and the induction period is at most 20 seconds.

**8.** The process according to either of claims 6 and 7, wherein the acrylic acid used comprises at least 50 ppm of tocopherol and the Z% value of the resultant hydrogel-forming polymer capable of absorbing aqueous fluids is greater than 70.

**9.** The use of acid-functional monomers for preparing hydrogel-forming polymers capable of absorbing aqueous fluids, wherein the acid-functional monomers comprise tocopherol, especially alpha-tocopherol.

**10.** The use of acid-functional monomers according to claim 9 which comprise only tocopherol stabilizer.

**11.** The use of acrylic acid or acrylate as acid-functional monomer according to either of claims 9 and 10.

**12.** The use of hydrogel-forming polymers capable of absorbing aqueous fluids and obtainable by a process according to any of claims 6 to 8, for absorbing aqueous fluids, especially in hygiene articles.

## Revendications

**1.** Polymères formateurs d'hydrogel, absorbant des liquides aqueux, à base de polymères porteurs de groupes acides qui contiennent du tocophérol.

**2.** Polymères formateurs d'hydrogel, absorbant des liquides aqueux, suivant la revendication 1, dans lesquels le polymère porteur de groupes acides est un polyacrylate.

3. Polymères formateurs d'hydrogel, absorbant des liquides aqueux, suivant l'une des revendications 1 ou 2, dans lesquels le tocophérol se trouve réparti dans le polymère.

4. Polymères formateurs d'hydrogel, absorbant des liquides aqueux, suivant l'une des revendications 1 à 3, dans lesquels le tocophérol est de l'alpha-tocophérol.

5. Polymères formateurs d'hydrogel, absorbant des liquides aqueux, suivant l'une des revendications 1 à 4, dans lesquels le tocophérol est présent entre 10 et 1000 ppm, par rapport au monomère porteur de groupes acides.

6. Procédé de préparation de polymères formateurs d'hydrogel, absorbant des liquides aqueux, à base de polymères porteurs de groupes acides, dans lequel les monomères porteurs de groupes acides utilisés pour la préparation contiennent du tocophérol.

7. Procédé suivant la revendication 6, dans lequel l'acide acrylique utilisé contient au moins 50 ppm de tocophérol et la période d'induction est au maximum de 20 secondes.

8. Procédé suivant l'une des revendications 6 et 7, dans lequel l'acide acrylique utilisé contient au moins 50 ppm de tocophérol et la valeur Z % du polymère formateur d'hydrogel, absorbant des liquides aqueux, obtenu est supérieure à 70.

9. Utilisation de monomères porteurs de groupes acides pour la préparation de polymères formateurs d'hydrogel, absorbant des liquides aqueux, dans laquelle le monomère porteur de groupes acides contient du tocophérol, en particulier de l'alpha-tocophérol.

10. Utilisation de monomères porteurs de groupes acides suivant la revendication 9, dans laquelle le monomère porteur de groupes acides contient uniquement du tocophérol comme agent stabilisant.

11. Utilisation d'acide acrylique ou d'acrylate comme monomère porteur de groupes acides suivant l'une des revendications 9 et 10.

12. Utilisation de polymères formateurs d'hydrogel, absorbant des liquides aqueux, que l'on peut obtenir selon un procédé suivant l'une des revendications 6 à 8, pour l'absorption de liquides aqueux, en particulier dans des articles d'hygiène.